# EUROPEAN PATENT SPECIFICATION

(11) **EP 2 554 187 B1**
(45) Date of publication and mention of the grant of the patent: **08.11.2017**
(21) Application number: 10848739.8
(22) Date of filing: 08.09.2010
(51) Int. Cl.: A61L 27/04, A61L 27/56, B22F 3/11

(54) **METAL POROUS TANTALUM USED AS MEDICAL IMPLANT MATERIAL AND PREPARATION METHOD THEREOF**
PORÖSES METALLTANTALUM ZUR VERWENDUNG ALS MEDIZINISCHES IMPLANTATMATERIAL UND HERSTELLUNGSVERFAHREN DAFÜR
TANTALE POREUX MÉTALLIQUE UTILISÉ EN TANT QUE MATÉRIAU POUR IMPLANT MÉDICAL, ET SON PROCÉDÉ DE PRÉPARATION

(30) Priority: 31.03.2010 CN 201010136854
(43) Date of publication of application: 06.02.2013
(73) Proprietor: CHONGQING RUZER PHARMACEUTICAL CO., LTD., Yubei District Chongqing 401120 (CN)
(72) Inventor: RUAN, Jianming, Chongqing 401120 (CN); XIE, Jianquan, Chongqing 401120 (CN); JIE, Yunfeng, Chongqing 401120 (CN); WANG, Zhiqiang, Chongqing 401120 (CN); FENG, Hua, Chongqing 401120 (CN); YOU, Chao, Chongqing 401120 (CN); PANG, Qi, Chongqing 401120 (CN); ZHOU, Jian, Chongqing 401120 (CN); YE, Lei, Chongqing 401120 (CN); LIU, Bin, Chongqing 401120 (CN); ZHANG, Yazhuo, Chongqing 401120 (CN)
(74) Representative: Chaillot, Geneviève
(86) International application number: PCT/CN2010/076706
(87) International publication number: WO 2011/120280

(56) References cited:
- EP-A1- 2 149 414
- WO-A1-02/066693
- WO-A1-2006/029621
- WO-A2-2008/078164
- CN-A- 1 479 635
- CN-A- 1 658 914
- US-A1- 2003 153 981
- LIU CHAOHONG ET AL.: 'The Preparation of Porous beta -TCP Scaffold by Foam Impregnation and Its Biocompatibility' JOURNAL OF HEBEI UNIVERSITY OF TECHNOLOGY vol. 32, no. 6, December 2003, pages 17 - 20, XP008166604

## Description

### BACKGROUND OF THE INVENTION

### 1. Field of Invention

The present invention relates to a porous tantalum used for medical implantation and a method for preparing the same.

### 2. Description of the Related Art

A porous metal material used for medical implantation is important for specific application of treating traumatic osseous tissues, necrotic femoral tissues or the like. Such metal materials are normally porous stainless steel, porous titanium, and so on. As a porous implant material for the treatment of traumatic osseous tissues and necrotic femoral tissues, the porosity thereof should reach to 30-80%, and the pores should be all interconnected and well-distributed or partially interconnected depending on requirement. Thus, the porous implant material can make the growth phase of the osseous tissue uniform and have lower weight to fit the use of medical implantation.

Due to good biocompatibility and mechanical properties of the insoluble tantalum, the porous form thereof is potential in place of the traditional metal biomaterials mentioned above in order to be used as a medical implant material for the application of treating necrotic femoral tissues. Also, due to the harmlessness, non-toxicity, few of side effects, the rapid development of the medicine, and the further knowledge of tantalum as an implant material, the requirement of porous tantalum for medical implantation is getting more urgent than before, and the criterion of the quality of porous tantalum is getting much higher. As a porous tantalum for medical implantation, having a lot of well-distributed interconnecting pores and mechanical properties adaptable to human body are of great importance for being a connecting component to keep the newborn tissues growing well at the positions of traumatic osseous tissues or ossature defects.

The porous metal materials for medical implantation are manufactured mainly by powder sintering, like the preparation of general porous metal materials, especially by impregnating an organic foam body with metal powder and then sintering to obtain a porous metal having a foam structure with well-distributed interconnecting pores (also called "foam impregnation"). However, the porous metal materials with well-distributed interconnecting pores usually do not have sufficient mechanical properties because of the problems of the structural itself, as well as the collapse of the metal powder during sintering process. For now, such problems have not been solved according to any know research reports.

There are not many research subjects about the powder sintering process for making porous tantalum, especially few of papers has mentioned about the preparation of porous tantalum for medical implantation. CN Patent Publication No. 200510032174 discloses "Three-dimensional through-hole or part-hole interconnecting porous metal foam and its preparing method", and CN Patent Publication No. 200710152394 discloses "Porous foam tungsten and preparation method thereof''. Nevertheless, the porous metal is prepared for the applications of filtering materials, or for aerospace and other applications in high temperature environments. Furthermore, the porous metal processed in such application is not porous tantalum.

Regarding porous tantalum, US Patent No. 5282861 discloses "Open cell tantalum structures for cancellous bone implants and cell and tissue receptors". The porous tantalum is manufactured by commercial tantalum and a supporter such as a carbon skeleton obtained from heat degradation of polyurethane precursors. The carbon skeleton has multiple dodecahedrons with mesh structures inside and wholly distributed pores, and the porosity thereof reaches to 98%. Next, the commercial tantalum is bound to the carbon skeleton to form porous metal microstructure through chemical vapor deposition (CVD) (also called "chemical deposition"). The porous tantalum material obtained by such processes has a tantalum layer having 40-60µm of thickness, and has about 99 wt% of tantalum and about 1 wt% based on the weight of whole porous tantalum materials. The patent further discloses that the porous tantalum has 50-70MPa of compressive strength, 2.5-3.5GPa of elastic modulus, 63MPa of tensile strength and 15% of the amount of plastic deformation. However, the ductility of the porous tantalum described above is obviously insufficient causing subsequent processing of the porous tantalum, such as cutting the formed material. Similarly, the porous tantalum prepared by such methods mentioned above like foam impregnation has the same problems. Due to the limitation of the manufacturing methods, the obtained products usually are not pure enough and have remaining residues of the carbon skeleton such that the biosafety is much decreased."

WO 02/066693 A1 discloses a method for preparing a porous metal implementation material (in particular porous titanium). The method comprises the steps of providing a polymeric foam (PU), impregnating the foam in a slurry of metal particles and metal hydride (Ti hydride or Ta hydride), drying the impregnated foam body, pyrogenating the foam body and sintering the porous material under the existence of metal hydride, wherein the metal is preferably titanium as well as tantalum.

### SUMMARY OF THE INVENTION

The primary objective of the present invention is to provide a porous tantalum used for medical implantation with good biocompatibility and biosafety.

The secondary objective of the present invention is to provide a method for preparing the porous tantalum used for medical implantation. The porous tantalum made by such method has good biocompatibility and biosafety, and more importantly has good mechanical properties.

To achieve the primary objective, a porous tantalum used for medical implantation in accordance with the present invention is produced by foam impregnation and having a foam structure with three-dimensional interconnecting pores, wherein the foam structure has a foam skeleton and sintered tantalum particles located on the foam skeleton, and multiple sintering neck structures formed between the tantalum particles.

The porous tantalum used for medical implantation having such structural characteristics can completely meet the requirement of biocompatibility and biosafety, especially the foam skeleton is accumulated by sintered tantalum powder, and the sintered neck structure formed between the tantalum particles greatly improves the mechanical properties of such material like ductility and anti-bending strength. Additionally, the porous tantalum has lower than 0.5% of impurities through testing. The porous tantalum has well-distributed interconnecting pores, and has 2.0-11g/cm³ of density, 40-80% of porosity, 150-500µm of pore diameter, 2.0-4.6GPa of elastic modulus, 35-120MPa of yield strength, 40-170MPa of compressive strength, 150-300MPa of hardness, 9.4%-17.3% of the amount of plastic deformation, 65-72MPa of tensile strength 9.3-14.7% of percentage elongation. Accordingly, the mechanical properties of the porous tantalum, like the elastic modulus and the yield strength are also improved. According to the test of anti-bending, the fracture rate of the sintered neck structure formed between tantalum particles is less than 45%, the fracture rate of the interior of tantalum particles is larger than 55%, indicated that the structural reliability of the porous tantalum of the invention is good.

The foam structure substantially has three-dimensional interconnecting pores, in other words, a limited amount of three-dimensional disconnecting pores are allowed to exist therein, for example, about 1% of the three-dimensional disconnecting pores are allowed to exist therein and can be ignored.

As used herein, the term of "sintered neck structure" means that the structure formed by the powder heated and then binding together under high temperature, also usually called "sintering phenomenon".

As used herein, the term "sintering" means that the process of metallurgical binding of powder or particles under high temperature, usually performed at the melting point of the major ingredient through atomic mobilization.

Through microscopic observation, it is found that the sintering neck structure (or named "attachable neck") is growing during sintering, and causing changes of its properties. With the increasing sintering temperature, longer sintering time or appropriate control of the temperature and time during sintering, the sintering neck structure can grow gradually, the proportion of the sintering neck structure and the strength of the sintered body are both increase as well. That is, the sintering neck structure formed by partial tantalum particles also can achieve the objective of the present invention. The porous tantalum of the present invention is produced by sintering the tantalum powder having less than 43µm of average diameter and less than 0.1% of oxygen content therein, wherein it has 40-80% of porosity and 150-500µm of pore diameter, and the sintering neck structures formed between at least 50% of the tantalum particles. The sintering neck structures are formed between at least 80% of the tantalum particles. In one aspect, besides the appropriate tantalum powder, the foam impregnation is using polyurethane foam having 0.48-0.89mm (preferably 0.56-0.72mm) of pore diameter, 0.015-0.035g/cm³ (preferably 0.025g/cm³) of density and larger than 50° (preferably 50°-80°) of hardness as an organic foam body. Accordingly, using such materials described above is beneficial to the formation and processing of the structure of the porous tantalum of the present invention, and also makes the process efficient and maintains sufficient mechanic properties

To achieve the secondary objective, a method for preparing the porous tantalum used for medical implantation in accordance with the present invention comprises steps of:
(a) providing an organic binder, dispersant and tantalum powder;
(b) mixing the organic binder and the dispersant to form a solution and then mixing the solution and the tantalum powder to form tantalum slurry;
(c) providing an organic foam body, wherein the organic foam body has multiple pores;
(d) casting the tantalum slurry into the organic foam body and impregnating the casted organic foam body with the tantalum slurry until the pores of the organic foam body are filled with the tantalum slurry;
(e) drying the impregnated organic foam body with the tantalum slurry to remove the dispersant;
(f) degreasing the dried organic foam body to separating the dried tantalum slurry from the organic binder and the organic foam body in a protective environment of inert gas;
(g) vacuum sintering the dried tantalum slurry to obtain a porous sintered body, wherein the porous sintered body has a foam skeleton, sintered tantalum particles located on the foam skeleton, and multiple sintering neck structures formed between the tantalum particles; and
(h) vacuum annealing and treating the porous sintered body with normal post-treatments to obtain the porous tantalum.

The method for preparing the porous tantalum of present invention is practicable, overcomes the technical bias about sintering process of porous metal powder (metallurgy sintering), and maintains the mechanical properties of the porous tantalum with such sintered neck structure, as well as the improved ductility thereof. Also, the porous tantalum prepared by such method can be conveniently and effectively used for the application of surgery implantation of medical metal material, especially as a component for connecting the tissue at positions of traumatic osseous tissues or ossature defects. Furthermore, the organic binder, dispersant and the organic foam body are resolvable such that the porous tantalum has great biocompatibility and biosafety when being used for medical implantation.

The organic binder can be, but not limited to, polyvinyl alcohol (PVA), starch, ethyl cellulose, or other alternative substances, and preferably is polyvinyl alcohol. The dispersant can be, but not limited to, water, ethanol or the like, and preferably is water. The organic foam body can be, but not limited to, polyurethane foam body, polyether ester foam body, or other alternative substances. The tantalum powder has the tantalum powder has less than 43µm of average diameter and than 0.1% of oxygen content, the organic binder is polyvinyl alcohol, the dispersant is water, the organic foam body is a polyurethane foam body, and the impregnated organic foam body with the tantalum slurry is dried by vacuum drying to remove water, and the obtained porous tantalum has 40-80% of porosity and 150-500µm of pore diameter and the sintering neck structures formed between at least 50% of the tantalum particles.

In one aspect, the solution is a 2-8 wt% (preferably 4-5 wt%) polyvinyl alcohol solution made by dissolving polyvinyl alcohol in distilled water under heat. In one aspect, 6-9 weight parts (preferably 7 weight parts) of tantalum powder and 1 weight part of the 2-8 wt% polyvinyl alcohol solution are mixed homogeneously and agitated to form a pasty substance (i.e. tantalum slurry), the pasty substance is casted into the polyurethane foam body having 0.48-0.89 mm (preferably 0.56-0.72 mm) of pore diameter, 0.015-0.035g/cm³ (preferably 0.025g/cm³) of density and larger than 50° (preferably 50°-80°) of hardness.

It is beneficial to lowering the impurities and keeping good mechanical properties of the porous tantalum of the present invention by using the tantalum powder having less than 43µm of average diameter and less than 0.1% of oxygen content. Also, it is beneficial to keeping required porosity and pore diameter of the porous tantalum of the present invention by using the polyurethane foam body having 0.48-0.89 mm of pore diameter, 0.015-0.035g/cm³ of density and larger than 50° of hardness. The technical features of the present invention optimize the conditions during processing, make the obtained porous tantalum have biocompatibility and biosafety, and benefit the formation of the sintered neck structures.

In one aspect, the impregnated organic foam body with tantalum slurry is dried by vacuum drying under 10⁻² -1Pa of vacuity, and the dried organic foam body with tantalum slurry is degreased at 400-800°C in a protective environment (such as under 10⁻⁴-10⁻³ Pa of vacuity) and the dried organic foam body and the organic binder are separated from the dried tantalum slurry, and the dried tantalum slurry is sintered by vacuum sintering at 2000-2200°C under 10⁻⁴-10⁻³Pa of vacuity and keeping the temperature for 1-5 hours to obtain the porous sintered body. Alternatively, the protective environment can be supplied by employing inert gas in place of the vacuum environment. Then, the porous sintered body is annealed by vacuum annealing through keeping the temperature at 1000-1250°C for 1-4 hours under less than10⁻⁴-10⁻³Pa of vacuity.

In one aspect, the dried organic foam body with tantalum slurry is degreased to separate the dried organic foam body and the organic binder from the dried tantalum slurry by increasing the temperature to 400-800°C at a rate of 0.5-5°C/min in a protective environment of argon and keeping the temperature for 30-120min of time, and then the dried tantalum slurry is sintered to form a porous sintered body under less than 10⁻³Pa of vacuity by increasing the temperature from room temperature to 1200-1500°C at a rate of 10-20°C/min and keeping the temperature for 1-2 hours, and then increasing the temperature to 2000-2200°C at a rate of less than 20°C/min and keeping the temperature for at least 2-4 hours; after sintered, the porous sintered body is cooled down by decreasing the temperature to 800°C at a rate of 10-25°C/min by stages of which is for 30-90 minutes per stage, and then natural cooling; the porous sintered body is annealed under less than 10-4Pa of vacuity by increasing the temperature to 1000-1250°C at a rate of less than 30°C/min and keeping the temperature for 4-6 hours, then decreasing the temperature slowly then rapidlyto room temperature at a rate of 5-30°C/min by stages of which is less than 1.5-3 hours per stages.

Furthermore, in another aspect, the impregnated organic foam body with tantalum slurry is dried by vacuum drying at 60-100°C for 4-8 hours. The dried organic foam body with tantalum slurry is degreased by increasing the temperature to 600-800°C by stages in a protective environment of argon (purity: 99.9999%), The temperature during degreasing is increased from room temperature to 400°C at a rate of 1-5°C/min and kept for 30-60 minutes, and then increased from 400°C to 600-800°C at a rate of 0.5-1.5°C/min and kept for 60-120 minutes. The dried tantalum slurry is sintered to form a porous sintered body by vacuum sintering. The temperature during vacuum sintering is increased to 1200-1250°C at a rate of 10-15°C/min and kept for 30-60 minutes under 10⁻⁴-10⁻³Pa of vacuity, and increased to 1500°C at a rate of 10-20°C/min and kept for 30-60 minutes under 10⁻⁴-10⁻³Pa of vacuity, and then increased to 2000-2200°C at a rate of 6-20°C/min and kept for 120-240 minutes under 10⁻⁴-10⁻³Pa of vacuity. After sintered, under 10⁻⁴-10⁻³Pa of vacuity, the temperature is decreased to 1500-1600°C at a rate of 10-20°C/min and kept for 30-60 minute, and then decreased to 1200-1250°C at a rate of 12-20°C/min and kept for 60-90 minute, and decreased to 800°C at a rate of 10-20°C/min. Then, the porous sintered body is cooled naturally. After cooled, the porous sintered body was annealed by vacuum annealing. The temperature during vacuum annealing is increased to 1000-1250°C at a rate of 15-30°C/min and kept for 240-480 minute under 10⁻⁴-10⁻³Pa of vacuity, and then decreased to 1000°C at a rate of 5-10°C/min and kept for 90-180 minute under 10⁻⁴-10⁻³Pa of vacuity, and decreased to 800°C at a rate of 10-20°C/min and kept for 60-120 minute under lower than 10⁻⁴Pa of vacuity, and decreased to room temperature at a rate of 20-30°C/min under 10⁻⁴-10⁻³Pa of vacuity.

As such, the treatments, like vacuum drying and degreasing, are beneficial to lowering the impurities and improving biocompatibility, biosafety and mechanical properties of the porous tantalum of the present invention. The optimization of the organic foam body overcomes the difficulty of collapse of the foam skeleton during sintering. The optimization of the conditions of the sintering and annealing processes is favorable to making more the sintered neck structures formed between the tantalum particles located on the foam skeleton accumulated by sintered tantalum powder, and further bettering the mechanical properties of the porous tantalum such as ductility, and lowering the fracture rate of the sintered neck structure.

### BRIEF DESCRIPTION OF THE DRAWINGS

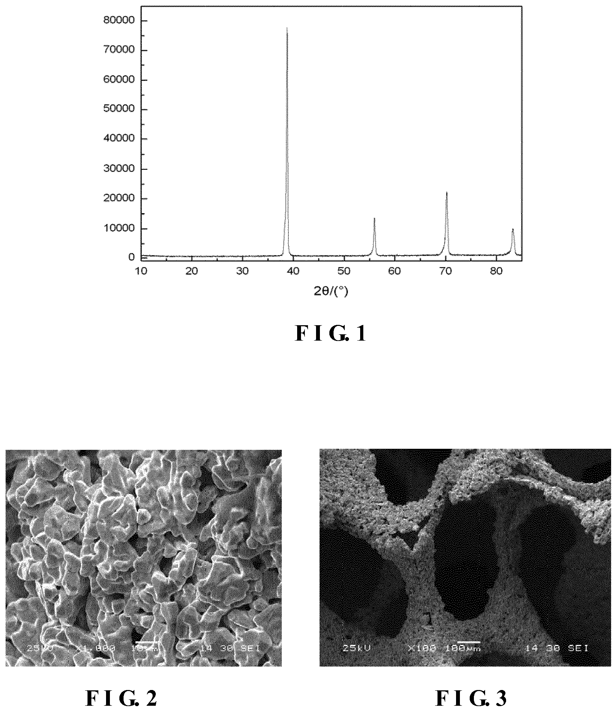

Fig. 1 is a powder x-ray diffraction (XRD) pattern of a porous tantalum used for medical implantation in accordance with the present invention.
Fig. 2 is a scanning electron microscopic (SEM) macrograph of a porous tantalum used for medical implantation in accordance with the present invention.
Fig. 3 is a scanning electron microscopic (SEM) micrograph of a porous tantalum used for medical implantation in accordance with the present invention.

It has been illustrated that:
1. The porous tantalum of the present invention has high porosity and the pores are well-distributed and interconnecting. Such three-dimensional interconnecting pores are advantageous to adhesion, differentiation and growth of osteocytes as well as ingrowths of bones, and also strengthen the connections between implants and bones to perform the biocompatibility well.
2. The porous tantalum of the present invention has excellent mechanical properties and microstructures with uniform particles and obvious sintered neck structures. The porous tantalum with such characteristics maintains good mechanical properties and ductility.

### DETAILED DESCRIPTION OF THE INVENTION

The present invention is more specifically described in the following paragraphs by reference to the drawings attached only by way of examples.

The present invention provides a porous tantalum used for medical implantation which is produced by foam impregnation. As shown in figures, the porous tantalum has a foam structure with three-dimensional interconnecting pores. The foam structure has a foam skeleton, and tantalum particles located on the foam skeleton, and multiple sintering neck structures formed between the tantalum particles. The present invention provides a porous tantalum used for medical implantation which is produced by sintering the tantalum powder having less than 43µm of average diameter and less than 0.1% of oxygen content, wherein it has 40-80% of porosity and 150-500µm of pore diameter, and the sintering neck structures formed between at least 50% of the tantalum particles.

In one preferred embodiment, the porous tantalum is produced by dissolving polyvinyl alcohol in distilled water under heat to form a polyvinyl alcohol solution with proper weight percentages of polyvinyl alcohol, mixing 1 weight part of the polyvinyl alcohol solution and adequate weight parts of tantalum powder to form tantalum slurry. The tantalum slurry is casted under pressure into a polyurethane foam body having multiple pores with adequate pore diameter, density and hardness, and then impregnated the casted polyurethane foam body with the tantalum slurry repeatedly until the pores of the polyurethane foam are filled with the tantalum slurry. The impregnated organic foam body with the tantalum slurry is dried at adequate temperature for an adequate time under adequate vacuity to remove water. The dried organic foam body is put into a tungsten device in an atmosphere furnace with increasing to adequate temperature. The dried organic foam body is degreased under constant temperature to separate the organic binder and the organic foam body from the dried tantalum slurry in a protective environment of inert gas and a degreased sample is formed (preferably of argon having at least 99.999% of purity). The inert gas is employed before increasing temperature to exclude the air in the atmosphere furnace before increasing the temperature. Then, the degreased sample is cooled naturally. The degreased sample in the tungsten device is heated in a sintering furnace and sintered under proper vacuity to form a sintered sample by increasing the temperature to 2000-2200°C by stages and keeping the temperature for 1-5 hours. The vacuity reaches to an adequate level before increasing the temperature in the sintering furnace. Then, the sintered sample is cooled naturally. The sintered sample is cooled down under constant vacuity, or by stages at a suitable rate of decreasing the temperature to keep the temperatures for proper periods of time. The inert gas can be employed as protective atmosphere during keeping the temperature. After cooled, the sintered sample is put in a corundum container in an annealing furnace with proper vacuity. The sintered sample is annealed by increasing the temperature by stages and keeping the temperatures for proper periods of time. The vacuity reaches to an adequate level before increasing the temperature in the annealing furnace. Then, the annealed sample is cooled naturally. The annealed sample is cooled down under constant vacuity, or by stages at a suitable rate of decreasing the temperature to keep the temperatures for proper periods of time. The inert gas can be employed as protective atmosphere during keeping the temperatures. The annealed sample is treated with normal post-treatments to obtain a porous tantalum.

The dried polyurethane foam body is put into a tungsten device in an atmosphere furnace and degreased by increasing to adequate temperature at a proper rate. The inert gas is employed before increasing temperature to exclude the air in the atmosphere furnace before increasing the temperature. The process of controlling temperature is increasing the temperature from room temperature to adequate temperature, keeping the temperature for a proper period of time, increasing the temperature again, and keeping the temperature for a proper period of time again. The degreased sample in the tungsten device is heated in a sintering furnace and sintered by increasing to the highest sintering temperature of tantalum at a proper rate in the sintering furnace under proper vacuity. The vacuity reaches to an adequate level before increasing the temperature in the sintering furnace. The temperature in the sintered furnace is increased to adequate temperature (such as 1200-1250°C) at a proper rate and kept for a proper period of time under constant vacuity, and then increased to adequate temperature (such as 1250-1500°C) at a proper rate and kept for a proper period of time under constant vacuity, and then increased to the highest sintering temperature of tantalum at a proper rate and kept for a proper period of time under constant vacuity. After sintered, the temperature is decreased to adequate temperature (such as 1500-1600°C) at a proper rate and kept for a proper period of time under constant vacuity, and then decreased to 800°C at a proper rate. Then, the sintered sample is cooled naturally. After cooled, the sintered sample is put into a corundum container in a annealing furnace under proper vacuity and annealed by increasing to adequate temperature (such as 1000-1250°C) at a proper rate. The vacuity reaches to an adequate level before increasing the temperature in the annealing furnace. The temperature in the sintered furnace is increased from room temperature to adequate temperature (such as 1000-1250°C) at a proper rate and kept for a proper period of time under constant vacuity, and then decreased to 1000°C at a proper rate and kept for a proper period of time, and then decreased to 800°C at a proper rate and kept for a proper period of time, and then decreased to room temperature. The annealed sample is treated with normal post-treatments to obtain a porous tantalum.

The sintering process used in the present invention is mainly the foam impregnation. It is analyzed and verified on a basis of lots of theories and experiments that the porous tantalum obtained by the sintering process has a foam structure with three-dimensional interconnecting pores (as shown in figures). The foam structure has a foam skeleton accumulated by the tantalum powder, and tantalum particles located in the foam skeleton, and sintered neck structures formed between the tantalum particles.

### Example 1:

12.5g of polyvinyl alcohol was put in a container filled in 240ml of water, and then the container was put on a hotplate. The polyvinyl alcohol and water are heated and agitated to form a polyvinyl alcohol solution. 60g of tantalum powder with less than 43µm of diameter and less than 0.1% of oxygen content was scaled by a 200g balance an added to 50ml of the polyvinyl alcohol solution (the polyvinyl alcohol solution was cooled). The tantalum powder and the polyvinyl alcohol solution were mix and agitated homogeneously to form tantalum slurry. The tantalum slurry was casted into a 10×10×30 mm porous polyurethane foam body (0.48mm of pore diameter, 0.025g/cm³ of density and 50° of hardness) until the pores of the polyurethane foam body were filled with the tantalum slurry. Then, the polyurethane foam body filled with the tantalum slurry was put into a porcelain dish placed in a vacuum drier. The polyurethane foam body filled with the tantalum slurry was dried in the vacuum drier at 60°C for 8 hours under 1Pa of vacuity. The dried polyurethane foam body filled with the tantalum slurry was degreased at 600°C for 120 minutes under lower than 10⁻⁴Pa of vacuity. The dried polyurethane foam and the dried tantalum slurry were separated after the process of degreasing. Then, the dried tantalum slurry are sintered in a vacuum sintering furnace at 2000°Cfor 2 hours under 10⁻⁴Pa of vacuity to form a porous sintered body. The argon is employed as a protective gas during sintering. The porous sintered body was cleaned out of the dust and dirt and then treated with normal post-treatments to obtain a porous tantalum.

With reference to the figures, it is shown that the porous tantalum made in Example 1 has a foam structure with three-dimensional interconnecting pores. The foam structure has a foam skeleton accumulated by the tantalum powder, and tantalum particles located in the foam skeleton, and multiple sintered neck structures formed between more than 50% of the tantalum particles.

The density, porosity, pore diameter and other mechanical properties of the obtained porous tantalum were tested by standard test methods such as GB/T5163-2006, GB/T5249-1985, GB/T6886-2001 and the like. The porous tantalum has three-dimensional interconnecting pores and less than 0.5% of impurities. The interconnecting pores are well-distributed. The tested porous tantalum has 3.5g/cm³ of density, higher than 40% of porosity, 100µm of average pore diameter, 2.0GPa of elastic modulus, 35MPa of yield strength, 40MPa of compressive strength, 150MPa of hardness, 17.3% of the amount of plastic deformation, 65MPa of tensile strength and 14.7% of percentage elongation. According to the anti-bending test on a basis of metal bending strength, the microstructure of the porous tantalum has less than 45% of fracture rate of the sintered neck structure, and larger than 55% of fracture rate of the interior of the tantalum particles.

### Example 2:

10g of polyvinyl alcohol was put in a container filled in 200ml of water, and then the container was put on a hotplate. The polyvinyl alcohol and water are heated and agitated to form a polyvinyl alcohol solution. 40g of tantalum powder with less than 43µm of diameter and less than 0.1% of oxygen content was scaled by a 200g balance an added to 32ml of the polyvinyl alcohol solution (the polyvinyl alcohol solution was cooled). The tantalum powder and the polyvinyl alcohol solution were mix and agitated homogeneously to form tantalum slurry. The tantalum slurry was casted into a 10×10×25 mm porous polyurethane foam body (0.56mm of pore diameter, 0.030g/cm³ of density and 60° of hardness) until the pores of the polyurethane foam body were filled with the tantalum slurry. Then, the polyurethane foam body filled with the tantalum slurry was put into a porcelain dish placed in a vacuum drier. The polyurethane foam body filled with the tantalum slurry was dried in the vacuum drier at 100°C for 4 hours under 10⁻²Pa of vacuity. The dried polyurethane foam body filled with the tantalum slurry was degreased at 800°C for 120 minutes under 10⁻⁴Pa of vacuity. The dried polyurethane foam body and the dried tantalum slurry were separated after the process of degreasing. Then, the dried tantalum slurry are sintered in a vacuum sintering furnace at 2100°C for 4 hours under 10⁻⁴Pa of vacuity and formed in a porous sintered body. The argon is employed as a protective gas during sintering. The porous sintered body was cleaned out of the dust and dirt and then treated with normal post-treatments to obtain a porous tantalum.

With reference to the figures, it is shown that the porous tantalum made in Example 2 has a foam structure with three-dimensional interconnecting pores. The foam structure has a foam skeleton accumulated by the tantalum powder, and tantalum particles located in the foam skeleton, and multiple sintered neck structures formed between more than 50% of the tantalum particles.

The density, porosity, pore diameter and other mechanical properties of the obtained porous tantalum were tested by standard test methods such as GB/T5163-2006, GB/T5249-1985, GB/T6886-2001 and the like. The porous tantalum has three-dimensional interconnecting pores and less than 0.5% of impurities. The interconnecting pores are well-distributed. The tested porous tantalum has 5g/cm³ of density, higher than 70% of porosity, 200µm of average pore diameter, 3.0GPa of elastic modulus, 70MPa of yield strength, 80MPa of compressive strength, 200MPa of hardness, 17% of the amount of plastic deformation, 70MPa of tensile strength and 14% of percentage elongation. According to the anti-bending test on a basis of metal bending strength, the microstructure of the porous tantalum has less than 40% of fracture rate of the sintered neck structure, and larger than 60% of fracture rate of the interior of the tantalum particles.

### Example 3:

11g of polyvinyl alcohol was put in a container filled in 220ml of water, and then the container was put on the hotplate. The polyvinyl alcohol and water are heated and agitated to form a polyvinyl alcohol solution. 45g of tantalum powder with less than 43µm of diameter and less than 0.1% of oxygen content was scaled by a 200g balance an added to 36ml of the polyvinyl alcohol solution (the polyvinyl alcohol solution was cooled). The tantalum powder and the polyvinyl alcohol solution were mix and agitated homogeneously to form tantalum slurry. The tantalum slurry was casted into a 8×8×25 mm porous polyurethane foam body (0.70mm of pore diameter, 0.035g/cm³ of density and 70° of hardness) until the pores of the polyurethane foam body were filled with the tantalum slurry. Then, the polyurethane foam body filled with the tantalum slurry was put into a porcelain dish placed in a vacuum drier. The polyurethane foam body filled with the tantalum slurry was dried in the vacuum drier at 80°C for 6 hours under 10⁻¹Pa of vacuity. The dried polyurethane foam filled with the tantalum slurry was degreased at 700°C for 90 minutes under 10⁻³Pa of vacuity. The dried polyurethane foam body and the dried tantalum slurry were separated after the process of degreasing. Then, the dried tantalum slurry are sintered in a vacuum sintering furnace at 2200°C for 2.5 hours under 10⁻³Pa of vacuity and formed in a porous sintered body. The argon is employed as a protective gas during sintering. The porous sintered body was cleaned out of the dust and dirt and then treated with normal post-treatments to obtain a porous tantalum.

With reference to the figures, it is shown that the porous tantalum made in Example 3 has a foam structure with three-dimensional interconnecting pores. The foam structure has a foam skeleton accumulated by the tantalum powder, and tantalum particles located in the foam skeleton, and multiple sintered neck structures formed between more than 50% of the tantalum particles.

The density, porosity, pore diameter and other mechanical properties of the obtained porous tantalum were tested by standard test methods such as GB/T5163-2006, GB/T5249-1985, GB/T6886-2001 and the like. The porous tantalum has three-dimensional interconnecting pores and less than 0.5% of impurities. The interconnecting pores are well-distributed. The tested porous tantalum has 8g/cm³ of density, higher than 60% of porosity, 300µm of average pore diameter, 4.0GPa of elastic modulus, 90MPa of yield strength, 100MPa of compressive strength, 250MPa of hardness, 17.2% of the amount of plastic deformation, 71MPa of tensile strength and 14.5% of percentage elongation. According to the anti-bending test on a basis of metal bending strength, the microstructure of the porous tantalum has less than 35% of fracture rate of the sintered neck structure, and larger than 65% of fracture rate of the interior of the tantalum particles.

### Example 4:

12g of polyvinyl alcohol was put in a container filled in 230ml of water, and then the container was put on a hotplate. The polyvinyl alcohol and water are heated and agitated to form a polyvinyl alcohol solution. 50g of tantalum powder with less than 43µm of diameter and less than 0.1% of oxygen content was scaled by a 200g balance an added to 40ml of the polyvinyl alcohol solution (the polyvinyl alcohol solution was cooled). The tantalum powder and the polyvinyl alcohol solution were mix and agitated homogeneously to form tantalum slurry. The tantalum slurry was casted into a 12×12×30 mm porous polyurethane foam body (0.60mm of pore diameter, 0.027g/cm³ of density and 80° of hardness) until the pores of the polyurethane foam body were filled with the tantalum slurry. Then, the polyurethane foam body filled with the tantalum slurry was put into a porcelain dish placed in a vacuum drier. The polyurethane foam body filled with the tantalum slurry was dried in the vacuum drier at 90°C for 5 hours under 1Pa of vacuity. The dried polyurethane foam body filled with the tantalum slurry was degreased at 500°C for 120 minutes under 10⁻⁴-10⁻³Pa of vacuity. The dried polyurethane foam body and the dried tantalum slurry were separated after the process of degreasing. Then, the dried tantalum slurry are sintered in a vacuum sintering furnace at 2150°Cfor 2 hours under 10⁻⁴Pa of vacuity and formed in a porous sintered body. The argon is employed as a protective gas during sintering. The porous sintered body was cleaned out of the dust and dirt and then treated by normal post-treatments to obtain a porous tantalum.

With reference to the figures, it is shown that the porous tantalum made in Example 4 has a foam structure with three-dimensional interconnecting pores. The foam structure has a foam skeleton accumulated by the tantalum powder, and tantalum particles located in the foam skeleton, and multiple sintered neck structure formed between more than 50% of the tantalum particles.

The density, porosity, pore diameter and other mechanical properties of the obtained porous tantalum were tested by standard test methods such as GB/T5163-2006, GB/T5249-1985, GB/T6886-2001 and the like. The porous tantalum has three-dimensional interconnecting pores and less than 0.5% of impurities. The interconnecting pores are well-distributed. The tested porous tantalum has 2.1-5.1g/cm³ of density, higher than 50% of porosity, 400µm of average pore diameter, 3.5GPa of elastic modulus, 120MPa of yield strength, 170MPa of compressive strength, 300MPa of hardness, 17.3% of the amount of plastic deformation, 72MPa of tensile strength and 14.6% of percentage elongation. According to the anti-bending test on a basis of metal bending strength, the microstructure of the porous tantalum has less than 43% of fracture rate of the sintered neck structure, and larger than 57% of fracture rate of the interior of the tantalum particles.

### Example 5:

Tantalum powder having less than 43µm of diameter and less than 0.1 % of the oxygen content as a raw material was mixed with a polyvinyl alcohol solution as a binder solution to form tantalum slurry. The tantalum slurry was casted into a polyurethane foam body. The polyurethane foam body with the tantalum slurry was dried, degreased, vacuum sintered, vacuum annealed and treated with normal post-treatments to obtain a porous tantalum.

In the exemplary embodiment, the poly urethane foam body has 0.56-0.72mm of pore diameter, 0.025g/cm³ of density and 50°-80° of hardness. The polyurethane foam body with the tantalum slurry was dried under 10⁻²-1Pa of vacuity to remove water. The dried polyurethane foam body and polyvinyl alcohol are separated from the dried tantalum slurry at 400-800°C of the temperature under 10⁻⁴-10⁻³Pa of vacuity or in a protective environment of inert gas with keeping the temperature for 30-120 minutes. The dried tantalum slurry was sintered at 2000-2200°C under 10⁻⁴-10⁻³Pa of vacuity and keeping the temperature for 1-5 hours. The argon or other alternative inert gas was employed as a protective gas when keeping the temperature during sintering process, to obtain a porous sintered body. After sintered, the porous sintered body was annealed by keeping the temperature at 1000-1250°Cfor 1-4 hours under 10⁻⁴-10⁻³Pa of vacuity, and then treated with normal post-treatments to obtain a porous tantalum.

With reference to the figures, it is shown that the porous tantalum made in Example 5 has the foam structure with three-dimensional interconnecting pores. The foam structure has a foam skeleton accumulated by tantalum powder, and tantalum particles located in the foam skeleton, and multiple sintered neck structures formed between more than 50% of the tantalum particles.

The density, porosity, pore diameter and other mechanical properties of the obtained porous tantalum were tested by standard test methods such as GB/T5163-2006, GB/T5249-1985, GB/T6886-2001 and the like. The porous tantalum has three-dimensional interconnecting pores and less than 0.5% of impurities. The interconnecting pores are well-distributed. The tested porous tantalum has 2.1-11g/cm³ of density, 40-80% of porosity, 150-500µm of average pore diameter, 2.0-4.6GPa of elastic modulus, 35-120MPa of yield strength, 40-170MPa of compressive strength, 150-300MPa of hardness, 9.4-17.3% of the amount of plastic deformation, 65-72MPa of tensile strength and 14.7% of percentage elongation. According to the anti-bending test on a basis of metal bending strength, the microstructure of the porous tantalum has less than 43% of fracture rate of the sintered neck structure, and larger than 57% of fracture rate of the interior of the tantalum particles.

### Example 6:

Tantalum powder having less than 43µm of diameter and less than 0.1 % of the oxygen content as a raw material was mixed with a polyvinyl alcohol solution as a binder solution to form tantalum slurry. The tantalum slurry was casted into a polyurethane foam body having 0.48-0.89mm of pore diameter, 0.015-0.035 g/cm³ and 50°-80° of hardness. The polyurethane foam body with the tantalum slurry was dried, degreased, vacuum sintered, vacuum annealed and treated with normal post-treatments to obtain a porous tantalum.

In the exemplary embodiment, polyvinyl alcohol was dissolved in the distilled water under heat to form a 5 wt% polyvinyl alcohol solution. 7 weight parts of tantalum powder and 1 weight part of the 5 wt% polyvinyl alcohol solution were mixed homogeneously and agitated to form pasty tantalum slurry. The polyurethane foam body was impregnated repeatedly until the pores of the polyurethane foam body were filled with the tantalum slurry. The polyurethane foam body with the tantalum slurry was dried at 60-100°C for 4-8 hours under 1Pa of vacuity to remove water.

The dried polyurethane foam body was put into a tungsten device in a non-oxidizing atmosphere furnace with increasing to 800°C at a proper rate. The dried organic foam body was degreased under argon having at least 99.999% of purity as a protective gas. The pure argon was employed as a protective gas for 30 minutes before increasing the temperature to exclude the air in the atmosphere furnace. The temperature was increased from room temperature to 400°C at a rate of 1°C/min with argon flowing at a rate of 0.5L/min and kept for 30 minutes, and then increased to 400-800°C at a rate of 0.5°C/min with argon flowing at a rate of 1L/min and kept for 120 minutes. Then, the power was closed and the degreased sample was cooled down with the temperature in the furnace while argon flowing at a rate of 1L/min. The argon supplier was closed until the temperature of the degreased sample was decreased to the room temperature.

The degreased sample in the tungsten device was heated in a sintering furnace and sintered by increasing to 2200°C at a proper rate. The vacuity reached to 10⁻⁴Pa before increasing the temperature in the sintering furnace. The temperature in the sintered furnace was increased from room temperature to 1200°C at a rate of 10-15°C/min and kept for 30 minutes under 10⁻⁴Pa of vacuity, and then increased to 1500°C at a rate of 10°C/min and kept for 30min under 10⁻⁴-10⁻³Pa of vacuity, and then increased to 2200°C at a rate of 6°C/min and kept for 120 minutes under 10⁻³Pa of vacuity. After sintered, under 10⁻³Pa of vacuity, the temperature was decreased to 1600°C at a rate of 10-15°C/min and kept for 30 minutes, and then decreased to 1200°C at a rate of 12°C/min and kept for 60min, and then decreased to 800°C at a rate of 10°C/min. Then, the sintered sample was cooled naturally.

After cooled, the sintered sample was put into the corundum container in an annealing furnace under proper vacuity and annealed by increasing to 1250°C at a proper rate. The vacuity reached to 10⁻⁴Pa before increasing the temperature in the annealing furnace. The temperature in the sintered furnace was increased from room temperature to 1250°C at a rate of 15°C/min and kept 240 minutes under 10⁻⁴-10⁻³Pa of vacuity, and then decreased to 1000°C at a rate of 5°C/min and kept for 180min under 10⁻⁴-10⁻³Pa of vacuity, and then decreased to 800°C at a rate of 10°C/min and kept for 120 minutes under 10⁻⁴Pa of vacuity, and then decreased to room temperature at a rate of 20°C/min under 10⁻⁴Pa of vacuity. Then, the annealed sample was treated with normal post-treatments to obtain a porous tantalum.

With reference to the figures, it is shown that the porous tantalum made in Example 6 has a foam structure with three-dimensional interconnecting pores. The foam structure has a foam skeleton accumulated by the tantalum powder, and tantalum particles located in the foam skeleton, and multiple sintered neck structures formed between more than 80% of the tantalum particles.

The density, porosity, pore diameter and other mechanical properties of the obtained porous tantalum were tested by standard test methods such as GB/T5163-2006, GB/T5249-1985, GB/T6886-2001 and the like. The porous tantalum has three-dimensional interconnecting pores and less than 0.5% of impurities. The interconnecting pores are well-distributed. The tested porous tantalum has 4.52g/cm³ of density, 72.8% of porosity, 300µm of average pore diameter, 3.5GPa of elastic modulus, 108MPa of yield strength, 69MPa of compressive strength, 230MPa of hardness, 11.4% of the amount of plastic deformation, 67MPa of tensile strength and 14.7% of percentage elongation. According to the anti-bending test on a basis of metal bending strength, the microstructure of the porous tantalum has less than 40% of fracture rate of the sintered neck structure, and larger than 60% of fracture rate of the interior of the tantalum particles.

Besides the examples illustrated above, the organic binder also can be starch, ethyl cellulose or other alternative substances, the dispersant also can be pure ethanol or other alternative substance, and the organic foam body also can be a polyether ester foam body. However, no matter what substances are used as the organic binder, the dispersant and the organic foam body, the purposes of their use are looking forward things that the dispersant can be easily removed and the metal powder during sintering process cannot collapse but easily eliminated to form the porous tantalum having a foam structure with three-dimensional interconnecting pores.

Besides the processes mentioned above in Example 6, people skilled in the art can make changes and modifications of the conditions to obtain the porous tantalum of the present invention.

**Table 1**

| Example | Diameter of tantalum powder (□m) /Oxygen content (%) | PVA(g) /Water (ml) | Tantalum powder (weight part) /PVA solution (weight part) | Pore diameter of polyurethane foam body (mm) | Density of polyurethane foam body (g/cm³) | Hardness of polyurethane foam body (°) |
|---|---|---|---|---|---|---|
| 7 | <3/<0.1 | 2/100 | 1/7.5 | 0.50 | 0.025 | 50 |
| 8 | <40/<0.1 | 3/100 | 1/8.5 | 0.88 | 0.035 | 55 |
| 9 | <42/<0.1 | 4/100 | 1/7.7 | 0.70 | 0.030 | 77 |
| 10 | <41/<0.1 | 5/100 | 1/8.3 | 0.60 | 0.027 | 66 |
| 11 | <39/<0.1 | 6/100 | 1/6.0 | 0.55 | 0.033 | 73 |
| 12 | <8/<0.1 | 7/100 | 1/7.3 | 0.56 | 0.016 | 63 |
| 13 | <35/<0.1 | 8/100 | 1/8.1 | 0.72 | 0.018 | 53 |

**Table 2**

| Example | Vacuity of drying (Pa) | The atmosphere of degreasing (Pa) | The atmosphere of sintering (Pa) | The atmosphere of annealing (Pa) |
|---|---|---|---|---|
| | /Temperature (°C) | | / Temperature (°C) | /The rate of increasing or decreasing the temperature (°C/min) |
| | /Time (h) | / Temperature (°C) | /Time (min) | |
| | | /Time (min) | | /Temperature (°C) |
| | | | | /Time of keeping temperature (min) |
| 7 | 1/65/6.5 | increasing from room temperature to 400°C at a rate of 1°C/min and keeping the temperature for 60min; | increasing from room temperature to 1200°C at a rate of 10°C/min and keeping the temperature for 60min under 10⁻⁴Pa of vacuity; | 10⁻⁴Pa |
| | | | | /increasing to 1030°C at a rate of 15°C/min and keeping the temperature for 480min |
| | | | | /decreasing to 1000°C at a rate of 5°C/min and keeping the temperature for 180min; |
| | | increasing from 400°C to 600°C at a rate of 0.5°C/min and keeping the temperature for 120min. | increasing to 1250°C at a rate of 11°C/min and keeping the temperature for 60min; | |
| | | | | /decreasing to 800°C at a rate of 11 °C/min and keeping the temperature for 108min; |
| | | | increasing to 2030°C at a rate of 6°C/min and keeping the temperature for 240min under 10⁻³Pa of vacuity; | / decreasing to room temperature at a rate of 21°C/min |
| | | | increasing from room temperature to 1200°C at a rate of 10°C/min and keeping the temperature for 60min under 10⁻⁴Pa of vacuity; | |
| | | | decreasing to 1520°C at a rate of 11°C/min and keeping the temperature for 60min under 10⁻⁴-10⁻³Pa of vacuity; | |
| | | | decreasing to 1200°C at a rate of 13°C/min and keeping the temperature for 90min; | |
| | | | decreasing to 800°C at a rate of 13°C/min, and natural cooling | |
| 8 | 1/75/5.5 | increasing from room temperature to 400°C at a rate of 1.5°C/min and keeping the temperature for 58min; | increasing to 1210°C at a rate of 11°C/min and keeping the temperature for 58min under 10⁻⁴Pa of vacuity; | 10⁻⁴Pa |
| | | | | /increasing to 1050°C at a rate of 17°C/min and keeping the temperature for 450min |
| | | | increasing to 1270°C at a rate of 12°C/min and keeping the temperature for 55min; | /decreasing to 1000°C at a rate of 6°C/min and keeping the temperature for 150min; |
| | | increasing from 400°C to 600°C at a rate of 0.6°C/min and keeping the temperature for 110min | | /decreasing to 800°C at a rate of 12°C/min and keeping the temperature for 102min; |
| | | | increasing to 2050°C at a rate of 8°C/min and keeping the temperature for 220min under 10⁻³Pa of vacuity; | |
| | | | | / decreasing to room temperature at a rate of 22°C/min |
| | | | decreasing to 1530°C at a rate of 12°C/min and keeping the temperature for 55min under 10⁻⁴-10⁻³Pa of vacuity; | |
| | | | decreasing to 1210°C at a rate of 14°C/min and keeping the temperature | |
| | | | for 85min; | |
| | | | decreasing to 800°C at a rate of 14°C/min and natural cooling. | |
| 9 | 1/55/7 | increasing from room temperature to 400°C at a rate of 2°C/min and keeping the temperature for 56min; | increasing to 1220°C at a rate of 12°C/min and keeping the temperature for 55min under 10⁻⁴Pa of vacuity; | 10⁻⁴Pa |
| | | | | /increasing to 1100°C at a rate of 20°C/min and keeping the temperature for 420min |
| | | | increasing to 1300°C at a rate of 13°C/min and keeping the temperature for 50min; | /decreasing to 1000°C at a rate of 7°C/min and keeping the temperature for 130min; |
| | | increasing from 400°C to 680°C at a rate of 0.7°C/min and keeping the temperature for 100min; | | |
| | | | | /decreasing to 800°C at a rate of 13°C/min and keeping the temperature for 96min; |
| | | | increasing to 2100°C at a rate of 10°C/min and keeping the temperature for 200min under 10⁻³Pa of vacuity; | |
| | | | | / decreasing to room temperature at a rate of 23°C/min |
| | | | decreasing to 1540°C at a rate of 13°C/min and keeping the temperature | |
| | | | for 50min under 10⁻⁴-10⁻³Pa of vacuity; decreasing to 1220°C at a rate of 15°C/min and keeping the temperature for 80min; | |
| | | | decreasing to 800°C at a rate of 15°C/min and natural cooling | |
| 10 | 1/45/7.5 | increasing from room temperature to 400°C at a rate of 2.5°C/min and keeping the temperature for 55min; | increasing to 1230°C at a rate of 13°C/min and keeping the temperature for 50min under 10⁻⁴Pa of vacuity; | 10⁻⁴Pa |
| | | | | /increasing to 1150°C at a rate of 22°C/min and keeping the temperature for 360min |
| | | | increasing to 1350°C at a rate of 14°C/min and keeping the temperature for 45min; | /decreasing to 1000°C at a rate of 8°C/min and keeping the temperature for 120min; |
| | | increasing from 400°C to 700°C at a rate of 0.8°C/min and keeping the temperature | | /decreasing to 800°C at a rate of 14°C/min and keeping the temperature for 90min; |
| | | | increasing to 2150°C at a rate of 12°C/min and keeping the temperature | |
| | | | | / decreasing to room temperature at a rate of |
| | | for 90min; | for 180min under 10⁻³Pa of vacuity; | 24°C/min |
| | | | decreasing to 1550°C at a rate of 14°C/min and keeping the temperature for 45min under 10⁻⁴-10⁻³Pa of vacuity; | |
| | | | decreasing to 1230°C at a rate of 16°C/min and keeping the temperature for 75min; | |
| | | | decreasing to 800°C at a rate of 16°C/min and natural cooling | |
| 11 | 1/85/6.0 | increasing from room temperature to 400°C at a rate of 3°C/min and keeping the temperature for 50min; | increasing to 1240°C at a rate of 14°C/min and keeping the temperature for 40min under 10⁻⁴Pa of vacuity; | 10⁻³Pa |
| | | | | /increasing to 1200°C at a rate of 25°C/min and keeping the temperature for 300min |
| | | | increasing to 1400°C at a rate of 15°C/min and keeping the temperature | /decreasing to 1000°C at a rate of 9°C/min and keeping the temperature for 100min; |
| | | increasing from 400°C to | | |
| | | 730°C at a rate of 0.9°C/min and keeping the temperature for 80min; | for 40min; | /decreasing to 800°C at a rate of 15°C/min and keeping the temperature for 84min; |
| | | | increasing to 2160°C at a rate of 14°C/min and keeping the temperature for 160min under 10⁻³Pa of vacuity; | |
| | | | | / decreasing to room temperature at a rate of 26°C/min |
| | | | decreasing to 1560°C at a rate of 15°C/min and keeping the temperature for 40min under 10⁻⁴-10⁻³Pa of vacuity; | |
| | | | decreasing to 1240°C at a rate of 17°C/min and keeping the temperature for 70min; | |
| | | | decreasing to 800°C at a rate of 17°C/min and natural cooling | |
| 12 | 1/95/5.5 | increasing from room temperature to 400°C at a | increasing to 1250°C at a rate of 15°C/min and keeping the temperature | 10⁻⁴Pa |
| | | | | /increasing to 1230°C at a rate of 27°C/min |
| | | rate of 4°C/min and keeping the temperature for 40min; | for 30min under 10⁻⁴Pa of vacuity; | and keeping the temperature for 270min |
| | | | increasing to 1450°C at a rate of 16°C/min and keeping the temperature for 35min; | /decreasing to 1000°C at a rate of 10°C/min and keeping the temperature for 90min; |
| | | increasing from 400°C to 750°C at a rate of 1.0°C/min and keeping the temperature for 70min; | | |
| | | | | /decreasing to 800°C at a rate of 16°C/min and keeping the temperature for 78min; |
| | | | increasing to 2170°C at a rate of 16°C/min and keeping the temperature for 140min under 10⁻³Pa of vacuity; | |
| | | | | / decreasing to room temperature at a rate of 27°C/min |
| | | | decreasing to 1570°C at a rate of 16°C/min and keeping the temperature for 35min under 10⁻⁴-10⁻³Pa of vacuity; | |
| | | | decreasing to 1245°C at a rate of 18°C/min and keeping the temperature for 65min; | |
| | | | decreasing to 800°C at a rate of | |
| | | | 18°C/min and natural cooling | |
| 13 | 1/100/4.5 | increasing from room temperature to 400°C at a rate of 2°C/min and keeping the temperature for 30min; | increasing to 1225°C at a rate of 13°C/min and keeping the temperature for 45 min under 10⁻⁴Pa of vacuity; | 10⁻⁴Pa |
| | | | | /increasing to 1250°C at a rate of 30°C/min and keeping the temperature for 240min |
| | | | increasing to 1500°C at a rate of 17°C/min and keeping the temperature for 30min; | /decreasing to 1000°C at a rate of 5.5°C/min and keeping the temperature for 170min; |
| | | increasing from 400°C to 800°C at a rate of 1.3°C/min and keeping the temperature for 60min; | | |
| | | | | /decreasing to 800°C at a rate of 17°C/min and keeping the temperature for 72min; |
| | | | increasing to 2180°C at a rate of 18°C/min and keeping the temperature for 120min under 10⁻³Pa of vacuity; | |
| | | | | / decreasing to room temperature at a rate of 29°C/min |
| | | | decreasing to 1580°C at a rate of 18°C/min and keeping the temperature for 30min under 10⁻⁴-10⁻³Pa of vacuity; | |
| | | | decreasing to 1250°C at a rate of | |
| | | | 19°C/min and keeping the temperature for 60min; | |
| | | | decreasing to 800°C at a rate of 19°C/min and | |

**Table 3 The mechanical properties of the porous tantalum prepared in Example 7-13**

| Example | 7 | 8 | 9 | 10 | 11 | 12 | 13 |
|---|---|---|---|---|---|---|---|
| Density (g/cm³) | 9.9 | 7.5 | 5.8 | 4.2 | 9.1 | 3.5 | 8.3 |
| Porosity (%) | 40 | 55 | 65 | 75 | 45 | 80 | 50 |
| Pore diameter (µm) | 170 | 290 | 480 | 380 | 330 | 210 | 430 |
| Elastic modulus (GPa) | 2.1 | 2.5 | 3.3 | 3.9 | 3.6 | 4.3 | 4.5 |
| Yield strength (MPa) | 110 | 100 | 90 | 80 | 70 | 65 | 40 |
| Compressive strength (MPa) | 50 | 160 | 150 | 130 | 100 | 140 | 110 |
| Hardness (MPa) | 160 | 170 | 210 | 230 | 280 | 290 | 270 |
| The amount of plastic deformation (%) | 17.30 | 17.20 | 17.25 | 17.22 | 17.28 | 17.29 | 17.30 |
| Tensile strength (MPa) | 65 | 66 | 67 | 68 | 69 | 70 | 71 |
| Percentage elongation (%) | 14.70 | 14.60 | 14.50 | 14.65 | 14.55 | 14.75 | 14.80 |
| Fracture rate of the sintering neck structures (%) | 45 | 40 | 35 | 30 | 43 | 37 | 32 |
| Fracture rate of the interior of tantalum particles (%) | 55 | 60 | 65 | 70 | 57 | 63 | 68 |

## Claims

1. A porous tantalum used for medical implantation, produced by foam impregnation and having a foam structure with three-dimensional interconnecting pores, wherein the foam structure has a foam skeleton, tantalum particles located on the foam skeleton, and multiple sintering neck structures formed between the tantalum particles, wherein said porous tantalum is produced by sintering the tantalum powder having an average diameter of less than 43µm and oxygen content in an amount of less than 0.1%, wherein it has 40-80% of porosity and 150-500µm of pore diameter, and the sintering neck structures are formed between at least 50% of the tantalum particles.

2. The porous tantalum used for medical implantation as claimed in claim 1, wherein a polyurethane foam body having 0.48-0.89mm of pore diameter, 0.015-0.035g/cm³ of density and larger than 50° of hardness is used as an organic foam body in the foam impregnation.

3. The porous tantalum used for medical implantation as claimed in claim 2, wherein a polyurethane foam body having 0.56-0.72mm of pore diameter, 0.025g/cm³ of density and 50°-80° of hardness is used as an organic foam body in the foam impregnation.

4. A method for preparing the porous tantalum used for medical implantation as claimed in claim 1, which is made by foam impregnation, and comprising steps of:
(a) providing an organic binder, dispersant and tantalum powder;
(b) mixing the organic binder and the dispersant to form a solution and then mixing the solution and the tantalum powder to form tantalum slurry;
(c) providing an organic foam body, wherein the organic foam body has multiple pores;
(d) casting the tantalum slurry into the organic foam body and impregnating the casted organic foam body with the tantalum slurry until the pores of the organic foam body are filled with the tantalum slurry;
(e) drying the impregnated organic foam body with the tantalum slurry to remove the dispersant;
(f) degreasing the dried organic foam body to separate the dried tantalum slurry from the organic binder and the organic foam body in a protective environment of inert gas;
(g) vacuum sintering the dried tantalum slurry to obtain a porous sintered body, wherein the porous sintered body has a foam skeleton, sintered tantalum particles located on the foam skeleton, and multiple sintering neck structures formed between the tantalum particles; and
(h) vacuum annealing and treating the porous sintered body with normal post-treatments to obtain the porous tantalum.

5. The method as claimed in claim 4, wherein the organic binder is polyvinyl alcohol, the dispersant is water, the organic foam body is a polyurethane foam body, and the impregnated organic foam body with the tantalum slurry is dried by vacuum drying to remove water.

6. The method as claimed in claim 5, wherein the solution is a 2-8 wt% polyvinyl alcohol solution made by dissolving polyvinyl alcohol in distilled water under heat; 6-9 weight parts of tantalum powder and 1 weight part of the 2-8 wt% polyvinyl alcohol solution are mixed homogeneously and agitated to form the tantalum slurry, and the tantalum slurry is casted into the polyurethane foam body having 0.48-0.89 mm of pore diameter, 0.015-0.035g/cm³ of density and larger than 50° of hardness.

7. The method as claimed in claim 6, wherein the polyvinyl alcohol solution is 4-5 wt% polyvinyl alcohol solution; 7 weight parts of tantalum powder and 1 weight part of the 4-5 wt% polyvinyl alcohol solution are mixed homogeneously and agitated to form the tantalum slurry, the tantalum slurry is casted into the polyurethane foam body having 0.56-0.72 mm of pore diameter, 0.025g/cm³ of density and 50°-80° of hardness under pressure until the pores of the polyurethane foam are filled.

8. The method as claimed in any claim of claim 4 to 7, wherein the impregnated organic foam body is dried by vacuum drying under 10⁻² -1Pa of vacuity, and the dried organic foam body with the tantalum slurry is degreased at 400-800°C of the temperature in a protective environment of inert gas or under 10⁻⁴-10⁻³ Pa of vacuity, and the dried tantalum slurry is sintered by vacuum sintering under 10⁻⁴-10⁻³Pa of vacuity at 2000-2200°C and keeping the temperature for 1-5 hours in an environment of inert gas, and the porous sintered body is annealed by vacuum annealing through keeping the temperature at 1000-1250°C under 10⁻⁴-10⁻³Pa of vacuity and keeping the temperature for 1-4 hours.

9. The method as claimed in claim 8, wherein the dried organic foam body with tantalum slurry was degreased by increasing the temperature to 400-800°C at a rate of 0.5-5°C/min in a protective environment of argon and keeping the temperature for 30-120 minutes, and the dried tantalum slurry is sintered to form a porous sintered body under less than 10⁻³Pa of vacuity by increasing the temperature from room temperature to 1200-1500°C at a rate of 10-20°C/min and keeping the temperature for 1-2 hours, and then increasing the temperature from room temperature to 2000-2200°C at a rate of less than 20°C/min and keeping the temperature for at least 2-4 hours; after sintered, decreasing to 800°C the temperature at a rate of between 10-25°C/min by stages of which is 30-90 minutes per stage and then to room temperature with the temperature in the sintering furnace, and the porous sintered body is cooled down; then after cooled, the porous sintered body is annealed under less than 10⁻⁴Pa of vacuity by increasing the temperature to 1000-1250°C at a rate of less than 30°C/min and keeping the temperature for 4-6 hours, and then decreasing the temperature slowly then rapidly to room temperature at a rate of 5-30°C/min by stages of which is less than 1.5 -3 hours per stages.

10. The method as claimed in claim 8, wherein the impregnated organic foam body with tantalum slurry was dried under adequate vacuity at 60-100°Cof the temperature for 4-8 hours, and the dried organic foam body with the tantalum slurry was degreased by increasing the temperature to 400-800°C by stages in a protective environment of argon; wherein t the dried organic foam body with the tantalum slurry was degreased by increasing the temperature from room temperature to 400°C at a rate of 1-5°C/min and keeping the temperature for 30-60 minutes, and then increasing the temperature from 400°C to 600-800°C at a rate of 0.5-1.5°C/min and keeping the temperature for 60-120 minutes; the dried tantalum slurry was sintered to form a porous sintered body by increasing the temperature to a range of 1200-1250°C at a rate of 10-15°C/min and keeping the temperature for 30-60 minutes under 10⁻⁴-10⁻³ Pa of vacuity, and then increasing the temperature to 1500°C at a rate of 10-20°C/min and keeping the temperature for 30-60 minutes under 10⁻⁴-10⁻³Pa of vacuity, and then increasing the temperature to a range of 2000-2200°C at a rate of 6-20°C/min and keeping the temperature for 120-240 minutes under 10⁻⁴-10⁻³Pa of vacuity; then after sintered, under 10⁻⁴-10⁻³Pa of vacuity, the porous sintered body was cooled down by decreasing the temperature to 1500-1600°C at a rate of 10-20°C/min and keeping the temperature for 30-60 minute, and then decreasing the temperature to 1200-1250°C at a rate of 12-20°C/min and keeping the temperature for 60-90 minutes, and then decreasing the temperature to 800°C at a rate of 10-20°C/min, and the porous sintered body is cooled down with the temperature in the furnace; then after cooled, the porous sintered body is annealed by increasing the temperature to 1000-1250°C at a rate of 15-30°C/min and keeping the temperature for 240-480 minute under 10⁻⁴-10⁻³Pa of vacuity, and then decreasing the temperature to 1000°C at a rate of 5-10°C/min and keeping the temperature for 90-180 minute under 10⁻⁴-10⁻³Pa of vacuity, and decreasing the temperature to 800°C at a rate of 10-20°C/min and keeping the temperature for 60-120 minute under lower than 10⁻⁴Pa of vacuity, and decreasing to room temperature at a rate of 20-30°C/min under 10⁻⁴-10⁻³Pa of vacuity.

11. The method as claimed in claim 10, wherein the dried organic foam body with tantalum slurry is degreased by increasing the temperature to 400°C with argon flowing at a rate of 0.5L/min and keeping the temperature for 30 minutes, and then increasing the temperature to 400-600°C with argon flowing at a rate of 1L/min and keeping the temperature for 120 minutes, wherein the purity of argon is 99.9999%; the dried tantalum slurry is sintered by increasing the temperature to 1200°C from room temperature at a rate of 10-15°C/min and keeping the temperature for 30 minutes under 10⁻⁴Pa of vacuity, and then increasing the temperature to 1500°C a rate of 10°C/min and keeping the temperature for 30 minutes, and increasing the temperature to 2200°C a rate of 6°C/min and keeping the temperature for 120 minutes under 10⁻³Pa of vacuity; then after sintered, under 10⁻³Pa of vacuity, the porous sintered body is cooled down under 10⁻³Pa of vacuity by decreasing the temperature to 1600°C and keeping the temperature for 30 minutes, and decreasing the temperature to 1200°C at a rate of 12°C/min and keeping the temperature for 60 minutes, and decreasing the temperature to 800°C at a rate of 10°C/min, and the porous sintered body is cooled down with the temperature in the furnace; after cooled, the porous sintered body is annealed by vacuum annealing by increasing the temperature to 1250°C at a rate of 15°C/min and keeping the temperature for 240 minute under 10⁻⁴-10⁻³ Pa of vacuity, and then decreasing the temperature to 1000°C at a rate of 5°C/min and keeping the temperature for 180 minute under 10⁻⁴-10⁻³Pa of vacuity, and then decreasing the temperature to 800°C at a rate of 10°C/min and keeping the temperature for 120 minute under 10⁻⁴Pa of vacuity, and decreasing the temperature to room temperature at a rate of 20°C/min under 10⁻⁴Pa of vacuity.

## Patentansprüche

1. Poröses Tantal für medizinische Implantate, hergestellt durch Schaumimprägnierung, mit einer Schaumstruktur mit dreidimensional verknüpften Poren, wobei die Schaumstruktur ein Schaumskelett hat, Tantalteilchen auf dem Schaumskelett positioniert sind und mehrere Sinter-Halsstrukturen zwischen den Tantalteilchen gebildet sind, wobei das poröse Tantal durch Sintern des Tantalpulvers hergestellt wird, das einen mittleren Durchmesser von weniger als 43 µm und einen Sauerstoffgehalt von weniger als 0,1% hat, mit 40-80% Porosität und 150-500 µm Porendurchmesser, und die Sinter-Halsstrukturen sind zwischen mindestens 50% der Tantalteilchen gebildet.

2. Poröses Tantal für medizinische Implantate nach Anspruch 1, wobei ein Polyuräthanschaumkörper mit 0,48-0,89 mm Porendurchmesser, 0,015-0,035 g/cm³ Dichte und mehr als 50° Härte als organischer Schaumkörper bei der Schaumimprägnierung benutzt wird.

3. Poröses Tantal für medizinische Implantate nach Anspruch 2, wobei ein Polyuräthanschaumkörper mit 0,56-0,72 mm Porendurchmesser, 0,025g/cm³ Dichte und 50°-80° Härte als organischer Schaumkörper bei der Schaumimprägnierung benutzt wird.

4. Verfahren zur Herstellung des porösen Tantals für medizinische Implantate nach Anspruch 1, das durch Schaumimprägnierung hergestellt wird, mit folgenden Phasen:
(a) Bereitstellung eines organischer Bindemittels, Dispersionsmittels und Tantalpulvers;
(b) Mischung des organischen Bindemittels und des Dispersionsmittels, um eine Lösung zu bilden, und dann Vermischung der Lösung mit dem Tantalpulver, um Tantalschlicker zu bilden;
(c) Bereitstellung eines organischen Schaumkörpers, wobei der organische Schaumkörper vielfache Poren hat;
(d) Gießen des Tantalschlickers in den organischen Schaumkörper und Imprägnieren des gegossenen organischen Schaumkörpers mit dem Tantalschlicker, bis die Poren des organischen Schaumkörpers mit dem Tantalschlicker gefüllt sind;
(e) Trocknen des imprägnierten organischen Schaumkörpers mit dem Tantalschlicker, um das Dispersionsmittel zu entfernen;
(f) Entfetten des getrockneten organischen Schaumkörpers, um den getrockneten Tantalschlicker von dem organischen Bindemittel und dem organischen Schaumkörper in einer Edelgasschutzumgebung zu trennen;
(g) Vakuumsintern des getrockneten Tantalschlickers, um einen porösen gesinterten Körper zu erhalten, wobei der poröse gesinterte Körper ein Schaumskelett hat, gesinterte Tantalteilchen auf dem Schaumskelett positioniert sind und mehrfache Sinter-Halsstrukturen zwischen den Tantalteilchen gebildet sind; und
(h) Vakuumglühen und Behandeln des porösen gesinterten Körpers mit normalen Nachbehandlungen, um das poröse Tantal zu erhalten.

5. Verfahren nach Anspruch 4, wobei das organische Bindemittel Polyvinylalkohol ist, das Dispersionsmittel Wasser und der organische Schaumkörper ein Polyuräthanschaumkörper ist, und der imprägnierte organische Schaumkörper mit dem Tantalschlicker wird durch Vakuumtrocknen getrocknet, um Wasser zu entfernen.

6. Verfahren nach Anspruch 5, wobei die Lösung eine 2-8 Gew.%-Polyvinylalkohollösung ist, hergestellt durch Auflösen von Polyvinylalkohol in destilliertem Wasser unter Hitze; 6-9 Gewichtsanteile Tantalpulver und 1 Gewichtsanteil der 2-8 Gew.%-Polyvinylalkohollösung werden homogen gemischt und geschüttelt, um den Tantalschlicker zu bilden, und der Tantalschlicker wird in den Polyuräthanschaumkörper gegossen, der 0,48-0,89 mm Porendurchmesser, 0,015-0,035g/cm³ Dichte und mehr als 50° Härte aufweist.

7. Verfahren nach Anspruch 6, wobei die Polyvinylalkohollösung eine 4-5 Gew.%-Polyvinylalkohollösung ist; 7 Gewichtsanteile Tantalpulver und 1 Gewichtsanteil der 4-5 Gew.%-Polyvinylalkohollösung werden homogen gemischt und geschüttelt, um den Tantalschlicker zu bilden, der Tantalschlicker wird in den Polyuräthanschaumkörper gegossen, der 0,56-0,72 mm Porendurchmesser, 0,025g/cm³ Dichte und 50°-80° Härte aufweist, unter Druck, bis die Poren des Polyuräthanschaums gefüllt sind.

8. Verfahren nach einem beliebigen der Ansprüche 4 bis 7, wobei der imprägnierte organische Schaumkörper durch Vakuumtrocknen bei 10⁻²-1 Pa Vakuum getrocknet wird, und der getrocknete organische Schaumkörper mit dem Tantalschlicker wird bei 400-800°C in einer Edelgasschutzumgebung entfettet oder bei 10⁻⁴-10⁻³ Pa Vakuum, und der getrocknete Tantalschlicker wird durch Vakuumsintern bei 10⁻⁴-10⁻³ Pa Vakuum bei 2000-2200°C und Halten der Temperatur 1-5 Stunden lang in einer Edelgasumgebung gesintert, und der poröse gesinterte Körper wird durch Vakuumglühen mit Halten der Temperatur bei 1000-1250°C bei 10⁻⁴-10⁻³ Pa Vakuum und Halten der Temperatur 1-4 Stunden lang geglüht.

9. Verfahren nach Anspruch 8, wobei der getrocknete organische Schaumkörper mit Tantalschlicker durch Erhöhung der Temperatur auf 400-800°C mit einer Rate von 0.5-5°C/min in einer Schutzumgebung von Argon bei Halten der Temperatur 30-120 Minuten lang entfettet wurde, und der getrocknete Tantalschlicker gesintert wird, um einen porösen gesinterten Körper mit unter 10⁻³Pa Vakuum zu bilden, durch Erhöhung der Temperatur von Raumtemperatur auf 1200-1500°C mit einer Rate von 10-20°C/min und Halten der Temperatur 1-2 Stunden lang, und dann Erhöhung der Temperatur von Raumtemperatur auf 2000-2200°C mit einer Rate von weniger als 20°C/min und Halten der Temperatur über mindestens 2-4 Stunden; nach dem Sintern, Senken der Temperatur auf 800°C mit einer Rate von 10-25°C/min in Phasen von 30-90 Minuten pro Phase und dann auf Raumtemperatur mit der Temperatur in dem Sinterofen, und der poröse gesinterte Körper wird heruntergekühlt; dann, nach der Abkühlung, wird der poröse gesinterte Körper bei unter 10⁻⁴ Pa Vakuum durch Erhöhung der Temperatur auf 1000-1250°C bei einer Rate von unter 30°C/min und Halten der Temperatur über 4-6 Stunden geglüht, und dann Senken der Temperatur langsam, dann schnell, auf Raumtemperatur bei einer Rate von 5-30°C/min in Phasen von unter 1,5 -3 Stunden pro Phase.

10. Verfahren nach Anspruch 8, wobei der imprägnierte organische Schaumkörper mit Tantalschlicker bei adäquatem Vakuum bei 60-100°C über 4-8 Stunden getrocknet wurde, und der getrocknete organische Schaumkörper mit dem Tantalschlicker wurde durch Erhöhung der Temperatur auf 400-800°C in Phasen in einer Argonschutzumgebung entfettet; wobei der getrocknete organische Schaumkörper mit dem Tantalschlicker durch Erhöhung der Temperatur von Raumtemperatur auf 400°C bei einer Rate von 1-5°C/min und Halten der Temperatur über 30-60 Minuten und dann Erhöhung der Temperatur von 400°C auf 600-800°C bei einer Rate von 0.5-1.5°C/min und Halten der Temperatur über 60-120 Minuten entfettet wurde; der getrocknete Tantalschlicker wurde gesintert, um einen porösen gesinterten Körper zu bilden, durch Erhöhung der Temperatur auf 1200-1250°C bei einer Rate von 10-15°C/min und Halten der Temperatur über 30-60 Minuten bei 10⁻⁴-10⁻³ Pa Vakuum, und dann Erhöhung der Temperatur auf 1500°C bei einer Rate von 10-20°C/min und Halten der Temperatur über 30-60 Minuten bei 10⁻⁴-10⁻³ Pa Vakuum, und dann Erhöhung der Temperatur auf 2000-2200°C bei einer Rate von 6-20°C/min und Halten der Temperatur über 120-240 Minuten bei 10⁻⁴-10⁻³ Pa Vakuum; dann, nach dem Sintern, bei 10⁻⁴-10⁻³ Pa Vakuum, wurde der poröse gesinterte Körper durch Verringerung der Temperatur auf 1500-1600°C bei einer Rate von 10-20°C/min und Halten der Temperatur über 30-60 Minuten heruntergekühlt, und dann Senken der Temperatur auf 1200-1250°C bei einer Rate von 12-20°C/min und Halten der Temperatur über 60-90 Minuten, und dann Senken der Temperatur auf 800°C bei einer Rate von 10-20°C/min, und der poröse gesinterte Körper wird mit der Temperatur in dem Ofen heruntergekühlt; dann, nach der Abkühlung, wird der poröse gesinterte Körper durch Erhöhung der Temperatur auf 1000-1250°C bei einer Rate von 15-30°C/min und Halten der Temperatur über 240-480 Minuten bei 10⁻⁴-10⁻³ Pa Vakuum geglüht, und dann Senken der Temperatur auf 1000°C bei einer Rate von 5-10°C/min und Halten der Temperatur über 90-180 Minuten bei 10⁻⁴-10⁻³ Pa Vakuum, und Senken der Temperatur auf 800°C bei einer Rate von 10-20°C/min und Halten der Temperatur über 60-120 Minuten bei unter 10⁻⁴ Pa Vakuum, und Senken auf Raumtemperatur bei einer Rate von 20-30°C/min bei 10⁻⁴-10⁻³ Pa Vakuum.

11. Verfahren nach Anspruch 10, wobei der getrocknete organische Schaumkörper mit Tantalschlicker durch Erhöhung der Temperatur auf 400°C mit Argon entfettet wird, das bei einer Rate von 0,5L/min und Halten der Temperatur über 30 Minuten fließt, und dann Erhöhung der Temperatur auf 400-600°C mit Argon, das bei einer Rate von 1L/min und Halten der Temperatur über 120 Minuten fließt, wobei die Argonreinheit 99,9999% beträgt; der getrocknete Tantalschlamm wird gesintert, indem man die Temperatur auf 1200°C von der Raumtemperatur erhöht, bei einer Rate von 10-15°C/min und Halten der Temperatur über 30 Minuten bei 10⁻⁴ Pa Vakuum, und dann Erhöhung der Temperatur auf 1500°C bei einer Rate von 10°C/min und Halten der Temperatur über 30 Minuten, und Erhöhung der Temperatur auf 2200°C bei einer Rate von 6°C/min und Halten der Temperatur über 120 Minuten bei 10⁻⁴ Pa Vakuum; dann, nach dem Sintern, bei 10^{-4 3}Pa Vakuum, wird der poröse gesinterte Körper bei 10⁻⁴ Pa Vakuum durch Verringerung der Temperatur auf 1600°C und Halten der Temperatur über 30 Minuten heruntergekühlt, und Senken der Temperatur auf 1200°C bei einer Rate von 12°C/min und Halten der Temperatur über 60 Minuten, und Senken der Temperatur auf 800°C bei einer Rate von 10°C/min, und der poröse gesinterte Körper wird mit der Temperatur in dem Ofen heruntergekühlt; nach der Abkühlung wird der poröse gesinterte Körper durch Vakuumglühen durch Erhöhung der Temperatur auf 1250°C bei einer Rate von 15°C/min und Halten der Temperatur über 240 Minuten bei 10⁻⁴-10⁻³ Pa Vakuum geglüht, und dann Senken der Temperatur auf 1000°C bei einer Rate von 5°C/min und Halten der Temperatur über 180 Minuten bei 10⁻⁴-10⁻³ Pa Vakuum, und dann Senken der Temperatur auf 800°C bei einer Rate von 10°C/min und Halten der Temperatur über 120 Minuten bei 10⁻⁴ Pa Vakuum, und Senken der Temperatur auf Raumtemperatur bei einer Rate von 20°C/min unter 10⁻⁴ Pa Vakuum.

## Revendications

1. Tantale poreux utilisé pour des implants médicaux, obtenu par imprégnation de mousse et ayant une structure de mousse avec des pores interconnectés tridimensionnels, dans lequel la structure de mousse a un squelette de mousse, des particules de tantale situées sur le squelette de mousse, et de multiples structures de col de frittage formées entre les particules de tantale, dans lequel ledit tantale poreux est obtenu par frittage de la poudre de tantale ayant un diamètre moyen de moins de 43 µm et une teneur en oxygène dans une quantité de moins de 0,1 %, dans lequel il a 40-80 % de porosité et 150-500 µm de diamètre de pore, et les structures de col de frittage sont formées entre au moins 50 % des particules de tantale.

2. Tantale poreux utilisé pour des implants médicaux, selon la revendication 1, dans lequel un corps de mousse de polyuréthane ayant 0,48-0,89 mm de diamètre de pore, 0,015-0,035 g/cm³ de masse volumique et plus de 50° de dureté est utilisé comme corps de mousse organique dans l'imprégnation de mousse.

3. Tantale poreux utilisé pour des implants médicaux, selon la revendication 2, dans lequel un corps de mousse de polyuréthane ayant 0,56-0,72 mm de diamètre de pore, 0,025 g/cm³ de masse volumique et 50°-80° de dureté est utilisé comme corps de mousse organique dans l'imprégnation de mousse.

4. Procédé de préparation du tantale poreux utilisé pour des implants médicaux selon la revendication 1, qui est obtenu par imprégnation de mousse, et comprenant les étapes de :
(a) se procurer un liant organique, un dispersant et de la poudre de tantale ;
(b) mélanger le liant organique et le dispersant pour former une solution, puis mélanger la solution et la poudre de tantale pour former une bouillie de tantale ;
(c) se procurer un corps de mousse organique, le corps de mousse organique ayant de multiples pores ;
(d) couler la bouillie de tantale dans le corps de mousse organique et imprégner le corps de mousse organique coulé par la bouillie de tantale jusqu'à ce que les pores du corps de mousse organique soient remplis par la bouillie de tantale ;
(e) sécher le corps de mousse organique imprégné par la bouillie de tantale pour retirer le dispersant ;
(f) dégraisser le corps de mousse organique séché pour séparer la bouillie de tantale séchée du liant organique et du corps de mousse organique dans un environnement protecteur de gaz inerte ;
(g) fritter sous vide la bouillie de tantale séchée pour obtenir un corps fritté poreux, le corps fritté poreux ayant un squelette de mousse, des particules de tantale frittées situées sur le squelette de mousse, et de multiples structures de col de frittage formées entre les particules de tantale ; et
(h) soumettre à un recuit sous vide et traiter le corps fritté poreux par des post-traitements normaux pour obtenir le tantale poreux.

5. Procédé selon la revendication 4, dans lequel le liant organique est l'alcool polyvinylique, le dispersant est l'eau, le corps de mousse organique est un corps de mousse de polyuréthane, et le corps de mousse organique imprégné par la bouillie de tantale est séché par séchage sous vide pour retirer l'eau.

6. Procédé selon la revendication 5, dans lequel la solution est une solution d'alcool polyvinylique à 2-8 % en poids, obtenue par dissolution d'alcool polyvinylique dans de l'eau distillée sous chauffage ; 6-9 parties en poids de poudre de tantale et 1 partie en poids de la solution d'alcool polyvinylique à 2-8 % en poids sont mélangées de façon homogène et agitées pour former la bouillie de tantale, et la bouillie de tantale est coulée dans le corps de mousse de polyuréthane ayant 0,48 - 0,89 mm de diamètre de pore, 0,015-0,035 g/cm³ de masse volumique et plus de 50° de dureté.

7. Procédé selon la revendication 6, dans lequel la solution d'alcool polyvinylique est une solution d'alcool polyvinylique à 4-5 % en poids ; 7 parties en poids de poudre de tantale et 1 partie en poids de la solution d'alcool polyvinylique à 4-5 % en poids sont mélangées de façon homogène et agitées pour former la bouillie de tantale, la bouillie de tantale est coulée dans le corps de mousse de polyuréthane ayant 0,56-0,72 mm de diamètre de pore, 0,025 g/cm³ de masse volumique et 50°-80° de dureté sous pression jusqu'à ce que les pores de la mousse de polyuréthane soient remplis.

8. Procédé selon l'une quelconque des revendications 4 à 7, dans lequel le corps de mousse organique imprégné est séché par séchage sous vide sous 10 ²-1Pa de vide, et le corps de mousse organique séché avec la bouillie de tantale est dégraissé à 400-800°C de température dans un environnement protecteur de gaz inerte ou sous 10⁻⁴-10⁻³ Pa de vide, et la bouillie de tantale séchée est frittée par frittage sous vide sous 10⁻⁴-10⁻³ Pa de vide à 2000-2200°C et maintien de la température pendant 1-5 heures dans un environnement de gaz inerte, et le corps fritté poreux est soumis à un recuit par recuit sous vide en maintenant la température à 1000-1250°C sous 10⁻⁴-10⁻³ Pa de vide et maintien de la température pendant 1-4 heures.

9. Procédé selon la revendication 8, dans lequel le corps de mousse organique séché avec la bouillie de tantale a été dégraissé par augmentation de la température jusqu'à 400-800°C à raison de 0,5-5°C/min dans un environnement protecteur d'argon et maintien de la température pendant 30-120 minutes, et la bouillie de tantale séchée est frittée pour former un corps fritté poreux sous moins de 10⁻³ Pa de vide par augmentation de la température de la température ambiante à 1200-1500°C à raison de 10-20°C/min et maintien de la température pendant 1-2 heures, puis augmentation de la température de la température ambiante à 2000-2200°C à raison de moins de 20°C/min et maintien de la température pendant au moins 2-4 heures ; après frittage, diminution jusqu'à 800°C de la température à raison d'entre 10-25°C/min par des stades de 30-90 minutes par stade puis jusqu'à la température ambiante avec la température dans le four de frittage, et le corps fritté poreux est refroidi ; puis après refroidissement, le corps fritté poreux est soumis à un recuit sous moins de 10⁻⁴ Pa de vide par augmentation de la température à 1000-1250°C à raison de moins de 30°C/min et maintien de la température pendant 4-6 heures, puis diminution de la température lentement puis rapidement jusqu'à la température ambiante à raison de 5-30°C/min par des stades de moins de 1,5-3 heures par stade.

10. Procédé selon la revendication 8, dans lequel le corps de mousse organique imprégné avec la bouillie de tantale a été séché sous vide adéquat à 60-100°C de température pendant 4-8 heures, et le corps de mousse organique séché avec la bouillie de tantale a été dégraissé par augmentation de la température à 400-800°C par stades dans un environnement protecteur d'argon ; dans lequel le corps de mousse organique séché avec la bouillie de tantale a été dégraissé par augmentation de la température de la température ambiante à 400°C à raison de 1-5°C/min et maintien de la température pendant 30-60 minutes, puis augmentation de la température de 400°C à 600-800°C à raison de 0,5-1,5°C/min et maintien de la température pendant 60-120 minutes ; la bouillie de tantale séchée a été frittée pour former un corps fritté poreux par augmentation de la température à une plage de 1200-1250°C à raison de 10-15°C/min et maintien de la température pendant 30-60 minutes sous 10⁻⁴-10⁻³ Pa de vide, puis augmentation de la température à 1500°C à raison de 10-20°C/min et maintien de la température pendant 30-60 minutes sous 10⁻⁴-10⁻³ Pa de vide, puis augmentation de la température à une plage de 2000-2200°C à raison de 6-20°C/min et maintien de la température pendant 120-240 minutes sous 10⁻⁴-10⁻³ Pa de vide ; puis après frittage, sous 10⁻⁴-10⁻³ Pa de vide, le corps fritté poreux a été refroidi par diminution de la température jusqu'à 1500-1600°C à raison de 10-20°C/min et maintien de la température pendant 30-60 minutes, puis diminution de la température jusqu'à 1200-1250°C à raison de 12-20°C/min et maintien de la température pendant 60-90 minutes, puis diminution de la température jusqu'à 800°C à raison de 10-20°C/min, et le corps fritté poreux est refroidi avec la température dans le four ; puis après refroidissement, le corps fritté poreux est soumis à un recuit par augmentation de la température à 1000-1250°C à raison de 15-30°C/min et maintien de la température pendant 240-480 minutes sous 10⁻⁴-10⁻³ Pa de vide, puis diminution de la température jusqu'à 1000°C à raison de 5-10°C/min et maintien de la température pendant 90-180 minutes sous 10⁻⁴-10⁻³ Pa de vide, et diminution de la température jusqu'à 800°C à raison de 10-20°C/min et maintien de la température pendant 60-120 minutes sous moins de 10⁻⁴ Pa de vide, et diminution jusqu'à la température ambiante à raison de 20-30°C/min sous 10⁻⁴-10⁻³ Pa de vide.

11. Procédé selon la revendication 10, dans lequel le corps de mousse organique séché avec la bouillie de tantale est dégraissé par augmentation de la température jusqu'à 400°C avec de l'argon s'écoulant à raison de 0,5 L/min et maintien de la température pendant 30 minutes, puis augmentation de la température jusqu'à 400-600°C avec de l'argon s'écoulant à raison de 1 L/min et maintien de la température pendant 120 minutes, la pureté d'argon étant de 99,9999 % ; la bouillie de tantale séchée est frittée par augmentation de la température jusqu'à 1200°C à partir de la température ambiante à raison de 10-15°C/min et maintien de la température pendant 30 minutes sous 10⁻⁴ Pa de vide, puis augmentation de la température jusqu'à 1500°C à raison de 10°C/min et maintien de la température pendant 30 minutes, et augmentation de la température jusqu'à 2200°C à raison de 6°C/min et maintien de la température pendant 120 minutes sous 10⁻³ Pa de vide; puis après frittage, sous 10⁻³ Pa de vide, le corps fritté poreux est refroidi sous 10⁻³ Pa de vide par diminution de la température jusqu'à 1600°C et maintien de la température pendant 30 minutes, et diminution de la température jusqu'à 1200°C à raison de 12°C/min et maintien de la température pendant 60 minutes, et diminution de la température jusqu'à 800°C à raison de 10°C/min, et le corps fritté poreux est refroidi avec la température dans le four ; après refroidissement, le corps fritté poreux est soumis à un recuit par recuit sous vide par augmentation de la température jusqu'à 1250°C à raison de 15°C/min et maintien de la température pendant 240 minutes sous 10⁻⁴-10⁻³ Pa de vide, puis diminution de la température jusqu'à 1000°C à raison de 5°C/min et maintien de la température pendant 180 minutes sous 10⁻⁴-10⁻³ Pa de vide, puis diminution de la température jusqu'à 800°C à raison de 10°C/min et maintien de la température pendant 120 minutes sous 10 Pa de vide, et diminution de la température jusqu'à la température ambiante à raison de 20°C/min sous 10⁻⁴ Pa de vide.
